Europäisches Patentamt

European Patent Office

Office européen des brevets

(19)

(11) Publication number: **0 034 034**
**B1**

(12)

# EUROPEAN PATENT SPECIFICATION

(45) Date of publication of patent specification: **05.10.83**

(51) Int. Cl.³: **A 23 L 1/30, A 23 L 1/34**

(21) Application number: **81300433.0**

(22) Date of filing: **03.02.81**

(54) **Nutrient composition.**

(30) Priority: **08.02.80 JP 14428/80**

(43) Date of publication of application:
**19.08.81 Bulletin 81/33**

(45) Publication of the grant of the patent:
**05.10.83 Bulletin 83/40**

(84) Designated Contracting States:
**DE FR GB IT**

(56) References cited:
**DE - A - 2 654 820**
**DE - A - 2 759 133**
**DE - A - 2 909 854**
**GB - A - 1 371 535**
**US - A - 4 144 357**

**Protein and amino acid functions (E. J. Bigwood) pp. 63 to 71**

**The file contains technical information submitted after the application was filed and not included in this specification**

(73) Proprietor: **AJINOMOTO CO., INC.**
**5-8, Kyobashi 1-chome, Chuo-ku**
**Tokyo 104 (JP)**

(72) Inventor: **Sato, Hiroshi**
**No. 969-1, Yahagi-cho**
**Chiba-shi Chiba-ken (JP)**
Inventor: **Ogoshi, Shohei**
**No. 3-2136, Kemigawa-cho**
**Chiba-shi Chiba-ken (JP)**
Inventor: **Inoue, Goro**
**No. 6-14, Yonban-cho Minamisaka**
**Tokushima Tokushima-ken (JP)**

(74) Representative: **Bond, Bentley George et al,**
**Haseltine Lake & Co. 28 Southampton Buildings**
**Chancery Lane**
**London WC2A 1AT (GB)**

Courier Press, Leamington Spa, England.

Nutrient composition

This invention relates to nutrient compositions, in particular to nutrient compositions for pediatrics.

Elemental dietary compositions comprising amino acids, carbohydrates such as monosaccharides and oligosaccharides, fats, vitamins, minerals and additives such as preservatives and emulsifiers for the nutrition of children are known. However, such compositions are not always suitable for children suffering from various symptoms or illnesses.

For example, children, particular weak children, often have irregular or poor metabolism of amino acids. In this case known nutrient compositions might not satisfy nutritional requirements.

According to the present invention, there is provided a nutrient composition containing amino acids, characterised in that the amino acids are the following amino acids in the following amounts:

| Amino acid | Parts by weight |
|---|---|
| L-isoleucine | 4.50 to 6.08 |
| L-leucine | 8.70 to 11.78 |
| L-lysine | 6.58 to 8.90 |
| L-methionine | 1.38 to 1.86 |
| L-cysteine | 1.87 to 2.53 |
| L-phenylalanine | 2.69 to 3.63 |
| L-tyrosine | 3.79 to 5.13 |
| L-threonine | 4.30 to 5.82 |
| L-tryptophan | 1.62 to 2.19 |
| L-valine | 4.65 to 6.29 |
| L-histidine | 2.43 to 3.29 |
| L-alanine | 8.32 to 11.26 |
| L-arginine | 6.00 to 8.12 |
| L-aspartic acid | 3.62 to 4.90 |
| L-glutamic acid | 6.47 to 8.76 |
| glycine | 1.96 to 2.66 |
| L-proline | 8.46 to 11.44 |
| L-serine | 7.66 to 10.36 |
| Total | 100 |

The composition may contain one more of carbohydrate, oil or fat fit for human consumption, vitamin and mineral.

The compositions of the invention may be particularly suitable for children for example children up to 6 or 10 years of age.

The numerical values for the amounts of the above amino acids relate to the acids in their free form. However, in the present invention, the amino acids can be used in the form of derivatives and adducts thereof (e.g. acid addition and base addition salts) which can be digested as amino acids in the human body. For example, mineral acid salts such as hydrochloric acid salts, organic acid salts, metal salts such as sodium salts, potassium salts, calcium salts and magnesium salts, peptides, N-acylated derivatives and hydrates, of the amino acids, and hydrates of the derivatives and adducts, may be employed in this invention. In this case, for such derivatives and adducts, the weights thereof are calculated as free amino acid, and such calculated values for free amino acid must fall within the ranges given above.

Amino acids such as lysine, cysteine, histidine and arginine can be used in the form of, for example, their hydrochloric acid salts or their acetic acid salts. Tyrosine can be used in the form of, for example, its ethyl ester hydrochloride or in the form of an N-acetylated derivative thereof, and aspartic acid can be used in the form of, for example, a metal salt thereof such as the potassium salt or the magnesium salt.

The content of amino acids in the composition of the present invention is usually 5 to 20% by weight.

Dextrin, for example, is employed as the carbohydrate used in the present invention. Also, monosaccharides and oligosaccharides can be employed. The carbohydrate is usually present in the composition in an amount of about 75 to 82%, but can be used greater amounts if necessary.

Oils of plants (such as soya bean, corn and cotton seed), for example, are employed as the fats used in the present invention. When low amounts of fat, for example, about 2 to 4% by weight, are used, the ability of the composition to dissolve or emulsify is improved, and furthermore occurrence of diarrhoea owing to a high fat content is reduced.

Vitamin A (e.g. retinole acetate), vitamin $B_1$ (e.g. thiamine hydrochloride), vitamin $B_2$ (e.g. sodium riboflavine phosphate), vitamin $B_6$ (e.g. pyridoxine hydrochloride), vitamin $B_{12}$ (e.g. cyanocobalamin),

vitamin C (e.g. ascorbic acid), vitamin $D_2$ (e.g. ergocalciferol), vitamin E (e.g. tocopherol acetate), vitamin $K_1$ (e.g. phytonadione), calcium pantothenic acid, nicotinamide, biotin, folic acid and choline bitartarate, for example, may be employed as vitamins in the present invention. Usually, about 50 to 100 mg of vitamin are contained in each 80 g of the nutrient composition. Such a concentration of vitamins is about 0.1%.

Vitamin C and vitamin $D_2$ in particular may be used in the compositions of the invention in much greater amounts than in known elemental dietary compositions when the compositions of the invention are to be used for the nutrition of young children. For example, desirably about 20 to 40 mg of ascorbic acid or about 5 to 15 $\mu$g of ergocalciferol is used for each 80 g of the nutrient composition.

Examples of minerals for use in the compositions of the invention are iron (e.g. as iron gluconic acid dihydrate), copper (e.g. as cupric sulphate pentahydrate), manganese (e.g. as manganese sulphate pentahydrate), zinc (e.g. as zinc sulphate heptahydrate), potassium (e.g. as potassium iodide or potassium chloride), sodium (e.g. as sodium citrate dihydrate), calcium (e.g. as calcium glycerophosphate) and mangesium (e.g. as magnesium sulphate heptahydrate). Usually about 3000 to 5000 mg of minerals are present in each 80 g of the nutrient composition. Such a concentration of minerals is about 2 to 8% by weight.

More desirably, iron and calcium are used in the compositions of the invention in amounts much more than in the known nutrient compositions or elemental dietary compositions when the compositions of the invention are to be used for the nutrition of young children. For example, desirably about 30 to 60 mg of iron gluconic acid dihydrate or about 1400 to 2000 mg of glycerophosphate is used for each 80 g of the nutrient composition of the invention.

When the nutrient composition of the present invention is a finished food, preservatives such as potassium sorbate and emulsifiers such as polysorbate and soya phosphatide as additives can be present in the compositions whereby, in the case of preservatives, invading microorganisms find it difficult to breed when the composition is in the form of a powder or solution, and whereby, in the case of emulsifiers, it is easy to dissolve or emulsify the compositions for smooth administration through the mouth or intestines.

When the nutrient compositions of the invention are administered to young children, it is preferably for the compositions to be in emulsified or homogenized form for ease of administration and digestion. The compositions can be administered through the intestines or through the mouth. When they are administered orally, they are usually employed in sticky state form (pasty material) or in the form of a solution, and they may be administered together with, for example, milk. Furthermore, the nutrient compositiosn may contain flavouring, seasoning or acidophilus drink additive, which help to make them more palatable for eating or drinking.

When the compositions are administered through the intestines via a tube they may be used in the form of, for example, a solution in water or warm water, which solution contains, for example, about 5 to 40% w/v of the composition.

The compositions according to the present invention can be used, for example, to improve various symptoms of sick children, to provide nutrition control before and after surgical operations, and to improve poor digestion and poor absorption of nourishment.

The invention is illustrated by the following Example.

Example

The following substances were homogeneously mixed in powder form:

| | |
|---|---|
| L-histidine monohydrochloride monohydrate | 0.380 g |
| L-isoleucine | 0.519 g |
| L-leucine | 1.004 g |
| L-lysine monohydrochloride | 0.949 g |
| L-methionine | 0.159 g |
| L-cysteine hydrochloride monohydrate | 0.313 g |
| L-phenylalanine | 0.310 g |
| N-acetyl-L-tyrosine | 0.538 g |
| L-threonine | 0.496 g |
| L-tryptophan | 0.186 g |
| L-valine | 0.537 g |
| L-alanine | 0.960 g |
| L-arginine | 0.693 g |
| Mixture of di-L-aspartic acid (monomagnesium salt) and L-aspartic acid (monopotassium salt) in the ratio 1:1 | 0.547 g |
| L-glutamic acid | 0.747 g |
| glycine | 0.227 g |
| L-proline | 0.976 g |
| L-serine | 0.883 g |
| dextrin | 62.88 g |

| | |
|---|---|
| soybean oil | 2.400 g |
| retinole acetate | 0.372 mg |
| thiamine hydrochloride | 0.323 mg |
| riboflavine phosphate (sodium salt) | 0.427 mg |
| pyridoxine hydrochloride | 0.445 mg |
| cyanocobalamin | 1.200 μg |
| ascorbic acid | 28.600 mg |
| ergocalciferol | 8.532 μg |
| tocopherol acetate | 5.507 mg |
| phytonadione | 14.667 mg |
| pantothenic acid (calcium salt) | 1.987 mg |
| nicotinamide | 3.667 mg |
| biotin | 65.333 μg |
| folic acid | 73.333 μg |
| choline bitartarate | 29.880 mg |
| gluconic acid dihydrate (iron salt) | 43.97 mg |
| cupric sulphate pentahydrate | 1.373 mg |
| manganese sulphate pentahydrate | 2.173 mg |
| zinc sulphate heptahydrate | 214.7 mg |
| potassium sorbate | 120 mg |
| "polysorbate 80" | 117.6 mg |
| soya phosphatide | 16.8 mg |

The above mixture was converted to a sticky form with water or dissolved in water for oral administration.

When it was administered through the intestines, 80 g of the above mixture was dissolved in warm water to give 300 ml of a solution (specific gravity=1.09, concentration=26.7% w/v) as a standard solution for administration.

Furthermore, solutions having various concentrations of the mixture in the range of from 5 to 40% w/v were produced and put into bags for administration. These solutions can be administered to children via the duodenum or jejunum, by the use of a fine catheter.

The nutrient compositions gave better results than known composition when administered to infants, particular sick infants.

**Claims**

1. A nutrient composition containing amino acids, characterised in that the amino acids comprise the following amino acids in the following amounts:

| Amino acid | Parts by weight |
|---|---|
| L-isoleucine | 4.50 to 6.08 |
| L-leucine | 8.70 to 11.78 |
| L-lysine | 6.58 to 8.90 |
| L-methionine | 1.38 to 1.86 |
| L-cysteine | 1.87 to 2.53 |
| L-phenylalanine | 2.69 to 3.63 |
| L-tyrosine | 3.79 to 5.13 |
| L-threonine | 4.30 to 5.82 |
| L-tryptophan | 1.62 to 2.19 |
| L-valine | 4.65 to 6.29 |
| L-histidine | 2.43 to 3.29 |
| L-alanine | 8.32 to 11.26 |
| L-arginine | 6.00 to 8.12 |
| L-aspartic acid | 3.62 to 4.90 |
| L-glutamic acid | 6.47 to 8.76 |
| glycine | 1.96 to 2.66 |
| L-proline | 8.46 to 11.44 |
| L-serine | 7.66 to 10.36 |
| Total | 100 |

2. A modification of a composition as claimed in claim 1, wherein one or more of said amino acids is in the form of a derivative or adduct thereof (the amount of said derivative or adduct being based upon the assumption that it is in the form of its free amino acid).

3. A composition as claimed in claim 1 or 2, wherein said amino acids (or derivative or adduct thereof) are present in the composition in a total amount of from 5 to 20% by weight.

4. A composition as claimed in claim 1, 2 or 3, wherein the composition contains one or more of carbohydrate, oil or fat, vitamin and mineral.

5. A composition as claimed in any of claims 1 to 4, wherein the composition contains one or more of preservative, emulsifier, flavouring, seasoning and water.

**Revendications**

1. Une composition alimentaire contenant des amino-acides, caractérisée en ce que les aminoacides comprennent les amino-acides suivants en les quantités suivantes:

| Aminoacide | Parties en poids |
|---|---|
| L-isoleucine | 4,5 à 6,08 |
| L-leucine | 8,70 à 11,78 |
| L-lysine | 6,58 à 8,90 |
| L-méthionine | 1,38 à 1,86 |
| L-cystéine | 1,87 à 2,53 |
| L-phénylalanine | 2,69 à 3,63 |
| L-tyrosine | 3,79 à 5,13 |
| L-thréonine | 4,30 à 5,82 |
| L-tryptophane | 1,62 à 2,19 |
| L-valine | 4,65 à 6,29 |
| L-histidine | 2,43 à 3,29 |
| L-alanine | 8,32 à 11,26 |
| L-arginine | 6,00 à 8,12 |
| Acide L-aspartique | 3,62 à 4,90 |
| Acide L-glutamique | 6,47 à 8,76 |
| Glycine | 1,96 à 2,66 |
| L-proline | 8,46 à 11,44 |
| L-sérine | 7,66 à 10,36 |
| Total | 100 |

2. Une modification d'une composition comme revendiqué dans la revendication 1, dans laquelle un ou plusieurs desdits aminoacides sont sous forme d'un dérivé ou d'un composé d'addition (la quantité dudit dérivé ou composé d'addition étant exprimée comme s'il était sous forme de son aminoacide libre).

3. Une composition comme revendiqué dans la revendication 1 ou 2, dans laquelle lesdits aminoacides (ou leurs dérivés ou produits d'addition) sont présents dans la composition en une quantité totale de 5 à 20% en poids.

4. Une composition comme revendiqué dans les revendications 1, 2 ou 3, dans laquelle la composition contient un ou plusieurs constituants choisis parmi les glucides, les huiles ou graisses, les vitamines et les substances minérales.

5. Une composition comme revendiqué dans l'une quelconque des revendications 1 à 4, dans laquelle la composition contient une ou plusieurs substances choisies parmi les conservateurs, les émulsifiants, les arômes, les assaisonnements et l'eau.

**Patentansprüche**

1. Aminosäuren enthaltende Nährpräparate, dadurch gekennzeichnet, daß sie die nachstehenden Aminosäuren in folgenden Mengenanteilen enthalten

| L-Isoleucin | 4,50 bis 6,08 Gew. Teile, |
|---|---|
| L-Leucin | 8,70 bis 11,78 Gew. Teile, |
| L-Lysin | 6,58 bis 8,90 Gew. Teile, |
| L-Methionin | 1,38 bis 1,86 Gew. Teile, |
| L-Cystein | 1,87 bis 2,53 Gew. Teile, |
| L-Phenylalanin | 2,69 bis 3,63 Gew. Teile, |
| L-Tyrosin | 3,79 bis 5,13 Gew. Teile, |
| L-Threonin | 4,30 bis 5,82 Gew. Teile, |
| L-Tryptophan | 1,62 bis 2,19 Gew. Teile, |
| L-Valin | 4,65 bis 6,29 Gew. Teile, |
| L-Histidin | 2,43 bis 3,29 Gew. Teile, |
| L-Alanin | 8,32 bis 11,26 Gew. Teile, |
| L-Arginin | 6,00 bis 8,12 Gew. Teile, |
| L-Asparaginsäure | 3,62 bis 4,90 Gew. Teile, |

5

| L-Glutaminsäure | 6,47 bis 8,76 Gew. Teile, |
| L-Glycin | 1,96 bis 2,66 Gew. Teile, |
| L-Prolin | 8,46 bis 11,44 Gew. Teile und |
| L-Serin | 7,66 bis 10,36 Gew. Teile |

bis zu einer Gesamtmenge von 100 Gew. Teilen.

2. Modifizierung der Präparate gemäß Anspruch 1, dadurch gekennzeichnet, daß eine oder mehrere der genannten Aminosäuren in Form eines Derivates oder Adduktes derselben vorliegt bzw. vorliegen, wobei die Menge des besagten Derivatives oder Addukts auf die Annahme bezogen wird, daß es in Form der freien Säure vorhanden ist.

3. Präparat gemäß den Ansprüchen 1 oder 2, dadurch gekennzeichnet, daß die genannten Aminosäuren oder die Derivate oder Adduktes derselben in dem Präparat in einer Gesamtmenge von 5 bis 20 Gew.-% vorhanden sind.

4. Präparat gemäß den Ansprüchen 1, 2 oder 3, dadurch gekennzeichnet, daß das Präparat ein oder mehrere Kohlehydrate, Öle oder Fette, Vitamine und Mineralstoffe enthält.

5. Präparat gemäß jedem der Ansprüche 1 bis 4, dadurch gekennzeichnet, daß das Präparat ein oder mehrere Konservierungsmittel, Emulgatoren, Aromastoffe, Gewürzstoffe und Wasser enthält.